Europäisches Patentamt

European Patent Office

Office européen des brevets

(11) Publication number: **0 295 063 B1**

# EUROPEAN PATENT SPECIFICATION

(45) Date of publication of patent specification: **25.08.93** (51) Int. Cl.⁵: **C12N 1/14**, C12R 1/645

(21) Application number: **88305196.3**

(22) Date of filing: **08.06.88**

(54) **Lignin-degrading microorganism having high activity and selectivity.**

(30) Priority: **09.06.87 JP 142347/87**
**09.06.87 JP 142348/87**

(43) Date of publication of application:
**14.12.88 Bulletin 88/50**

(45) Publication of the grant of the patent:
**25.08.93 Bulletin 93/34**

(84) Designated Contracting States:
**FR GB SE**

(56) References cited:
**US-A- 3 962 033**

**AGRIC. BIOL. CHEM., vol. 45, no. 3, 1981, pages 653-657; Y. IWAMURO et al.: "Effect of medium components and cultural conditions on the production of polysaccharide by Porodisculus pendulus and mycelium growth"**

(73) Proprietor: **KABUSHIKI KAISHA KOBE SEIKO SHO also known as Kobe Steel Ltd.**
**3-18 1-chome, Wakinohama-cho Chuo-ku**
**Kobe 651(JP)**

(72) Inventor: **Nishida, Tomoaki**
**4-17-509, Hoshigaoka 3-chome Tarumi-ku**
**Kobe-shi Hyogo-ken(JP)**
Inventor: **Kashino, Yoshinori**
**3-1, Shinoharaobanoyama-cho 2-chome**
**Nada-ku**
**Kobe-shi Hyogo-ken(JP)**
Inventor: **Takahara, Yoshimasa**
**3-20, Konan-cho Higashinada-ku**
**Kobe-shi Hyogo-ken(JP)**

(74) Representative: **Lewin, John Harvey et al**
**Elkington and Fife Prospect House 8 Pembroke Road**
**Sevenoaks, Kent TN13 1XR (GB)**

**Description**

BACKGROUND OF THE INVENTION

Field of the Invention

The present invention concerns novel microorganisms and more specifically it relates to novel microorganisms excellent in lignin-degrading activity and capable of selectively degrading lignin.

Accordingly, the present invention is generally utilized in the technical field of various wood processing industries such as wood saccharification and pulp and paper productions, as well as all of other fields for the degradation and/or molecular weight reduction of lignin.

Description of the Prior Art

Microbial degradation of lignin has been tried generally and it has been well known that those microorganisms belonging to white-rot fungi have such activity ("Encyclopedica Chemie" Vol. 9 p 589-590 published from Kyoritsu Shuppan Co.). Among them, Coriolus versicolor, Phanerochaete chrysosporium, etc have been noted as typical microorganisms and there have been used mainly in the field of research and development.

However, the activity of these strains described above is only such that about from 10 to 15 % of lignin-degradation is obtained during culture for several weeks and they have not yet been put to practical use because of their low activity. Thus, it has been impossible to conduct industrial treatment by using known strains.

Problem to be Solved by the Invention

As described above, existent white-rot fungi require a long period of time for the lignin-degradation and, in addition, their lignin-degrading activity is extremely insufficient. Furthermore, it has been impossible to utilize them to useful industrial use such as wood saccharification since they also degrade carbohydrates such as cellulose or hemicellulose.

SUMMARY OF THE INVENTION

Accordingly, it is an object of the present invention to provide novel microoraganims having extremely high lignin-degrading activity and capable of selectively degrading lignin.

The novel microorganisms according to the present invention include microorganism strain NK-1148,and strain NK-729W belonging to Porodisculus pendulus excellent in the lignin-degrading activity and capable of selectively degrading lignin.

DETAILED DESCRIPTION OF THE INVENTION AND PREFERRED EMBODIMENTS

The present invention has been made in view of the foregoing present technical situation. Although various methods of screening were repeated for existent microorganisms, aimed microorganisms could not be obtained.

Then, we have come up to a conclusion that, for obtaining microorganisms capable of overcoming the foregoing problems and, particularly, having excellent lignin-degrading activity and excellent in the selectivity for the lignin-degradation thereby being suitable to industrial application, the idea of merely screening existent microorganisms has to be changed drastically. It has keenly been considered that novel microorganisms which are not known so far can not but be isolated and screened for obtaining useful microorganisms industrially in order to attain the foregoing purpose.

Then, looking for the aimed novel microorganisms, the present invention at first collected sources for isolation of microorganisms from forest, as well as upland and native pasture in the country. Further, also referring to the screening method, we made earnest retrieval by using known methods, as well as a method invented by the present inventors (Japanese Patent Application No. Sho 61-281668) capable of effectively detecting the lignin-degrading microorganisms from specimens in the natural world such as decayed wood. That is, effective lignin-degrading microorganisms were isolated by the combination of massive collection of sources for isolation and new test method.

As a result, we have succeeded in isolation of novel microorganisms as the object of the present invention having extremely high lignin-degrading activity and capable of selectively degrading lignin, and have accomplished the present invention.

The newly isolated microorganisms obtained in this way have the following mycological properties.

The strain NK-1148 of the first invention has the following mycological characteristics.

## (1) State of growth in culture medium

| Culture medium | State of growth |
|---|---|
| Malt extract agar medium | +++ |
| Potato glucose agar medium | +++ |
| Czapek's agar medium | + |
| Sabouraud agar medium | ++ |
| Synthesized mucor agar medium | ++ |
| YpSs agar medium | +++ |
| Glucose dried yeast agar medium | +++ |

Note-1  Culture medium pH : 5.0 (before autoclave sterilization)

Note-2  Culture condition : 28°C, 7 day

Note-3  State of growth

weak    :  +

medium  :  ++

abundant :  +++

(2) Physiological and morphological properties

(1) pH range for growth (Potato glucose agar medium: cultured for 4 days at 28°C)
Grows at pH of about 3 - 9 and not grow at pH 2 and 10. Optimal pH: about 4 - 6.
(2) Temperature range for growth (Potato glucose agar medium: cultured for 4 days at pH 5)
Grows at a temperature of about 10 - 45°C, not grow at 50°C. Optimal temperature : about 28 - 37°C
(3) Phenol oxidase reaction (cultured for 4 days at 28°C)
Shows weak or negative.
(4) Morphology of colony (Potato glucose agar medium: cultured for 4 days at pH 5, 28°C)
White felt-like state.

Mycological properties such as physiological and morphological properties and growing state in the respective culture media were examined closely, but they were not identical with known microorganisms ever isolated and reported.

In addition, as apparent from examples to be described later, the microorganism of the present invention is particularly excellent in the lignin-degrading activity than the known microorganisms of Coriolus sp. and Phanerochaete sp., as well as outstanding selectivity has been recognized. Since such excellent lignin-degrading microorganism has not been present in the known microorganisms, we recognized the present microorganism as new one, named as strain NK-1148 and deposited it to Biseibutsu Kogyo Gijyutsu Kenkusho, of the Ministry of Industry and Technology, based on Budapest convention (Deposit No.: FERM BP-1859). Since there have been still several unknown features for the exact taxonomical criteria in the present microorganism, quite collect identification is difficult at present and will be made in the feature study.

Then, the strain NK-729W belonging to the microorganism of Porodisculus sp. of the second invention has the following mycological characteristics.

(1) State of growth in culture medium

| Culture medium | State of growth |
|---|---|
| Malt extract agar medium | +++ |
| Potato glucose agar medium | +++ |
| Czapek's agar medium | + |
| Sabouraud agar medium | ++ |
| Synthesized mucor agar medium | ++ |
| YpSs agar medium | +++ |
| Glucose dried yeast agar medium | +++ |

Note-1  Culture medium pH : 5.0 (before autoclave
        sterilization)

Note-2  Culture condition : 28°C,  7 day

Note-3  State of growth
            weak     :  +
            medium   :  ++
            abundant :  +++

(2) Physiological and morphological properties

(1) pH range for growth (Potato glucose agar medium: cultured for 4 days at 28°C)
Grows at pH of about 3 - 7 and not grow at pH 2 and 8. Optimal pH; about 4 - 5.
(2) Temperature range for growth (Potato glucose agar medium: cultured for 4 days at pH 5)
Grows at a temperature of about 10 - 32°C, not grow at 37°C. Optimal temperature : about 20 - 30°C
(3) Phenol oxidase reaction (cultured for 4 days at 28°C)
Positive nature activity.
(4) Morphology of colony (Potato glucose agar medium cultured for 4 days at pH 5, 28°C)
White hair-like state.

4

(5) Shape of fruit body

| | |
|---|---|
| Size : | 2 - 5 mm diameter |
| Shape : | Inverted cup shape (nose shape) |
| Edge or surface : | Edge turned inwardly, surface color of yellow black, having brown fleece or hair over the entire surface |
| Surface of tubular pore : | Pale white gray, recessed in a upturned dish shape, with small pore |
| Texture : | Soft leather-like texture, substantially white |

(6) Spore shape

About 3 - 4 x 1 μm, sausage-like shape, colorless and smooth.

In view of the mycological properties, the present microorganism is considered to belong to the microorganism of Porodisculus sp. and, among all, to Porodisculus pendulus.

However, as apparent from examples to be described later, the present microorganism not only shows outstanding lignin-degrading activity but also excellent selectivity. Since microorganism showing such excellent nature has not been present in known Porodisculus pendulus, we recognized the present microorganism as a new strain of Porodisculus pendulus, named it as Porodisculus pendulus NK-729W and deposited it to Biseibutsu Kogyo Gijyutsu Kenkyusho, of the Ministry of Industry and Technology based on Budapest convention (Deposit No. : FERM BP-1860).

For utilizing the present microorganisms to various wood processing industries, they may be treated in the same manner as usual having no particular considerations, therefore, which is also one of the excellent features of the present invention. For instance, when the present microorganisms are utilized for the degradation of lignin, the culture medium containing wood material may be inoculated with the present microorganisms and incubated at an appropriate temperature for a predetermined of time in the same manner as in other lignin-degrading microorganism. Since the present microorganisms have the outstanding property, they are particularly suitable to mass treatment and mass production using large-scaled vessels, the processing time can be extremely short and they are suitable to the industrial utilization. In addition, the present invention can be utilized generally also to wood saccharification industry, etc. and, since no delicate operations are required therefor, it is particularly suitable to industrial application, which is also one of the excellent features of the present invention.

Example 1

A culture medium prepared by adding 1.0 g of extractive-free beech wood-powder (60 - 80 mesh) and 2.5 ml of water to a 50 ml Erlenmeyer flask was sterilized at 120°C for 15 minutes. This medium was inoculated with NK-1148 strain (FERN BP-1859) according to the present invention and Coriolus versicolor IFO 30340 and Phanerochaete chrysosporium ATCC 34541 known so far as lignin-degrading microorganism as control, respectively, and cultured at 28°C for one month.

After cultivation, Klason lignin (JIS p8008-1961) and acid-soluble lignin (Chemistry of Lignin, edited by Junzo Nakano, p 53 (1982)) in wood powder were determined quantitatively. The weight loss was also measured to calculate the amount of carbohydrates. The result is shown in Table 1.

Table 1  : Result of wood-degradation

| Wood powder | | Before cultivation | After cultivation | | |
|---|---|---|---|---|---|
| | | | Novel microorga-nism of the invention | Coriolus versicolor | Phanerochaete chrysosporium |
| Weight loss | (g) | | 0.1603 | 0.0825 | 0.0452 |
| Klason lignin content | (g) | 0.2237 | 0.0970 | 0.1914 | 0.1998 |
| Acid-soluble lignin content | (g) | 0.0314 | 0.0363 | 0.0325 | 0.0367 |
| Total lignin content | (g) | 0.2551 | 0.1333 | 0.2239 | 0.2365 |
| Carbohydrate content | (g) | 0.7449 | 0.7064 | 0.6936 | 0.7183 |
| Klason lignin loss | (KL, %) | | 56.64 | 14.44 | 10.68 |
| Carbohydrate loss | (C, %) | | 5.17 | 6.89 | 3.57 |
| KL/C | | | 10.96 | 2.10 | 2.99 |

As apparent from the result in Table 1, the novel microoraganism according to the present invention is outstandingly excellent in the lignin-degrading activity and has extremely high selectivity.

Example 2

A culture medium prepared by adding 1.0 g of extractive-free beech wood-powder (60 - 80 mesh) and 2.5 ml of water to a 50 ml Erlenmeyer flask was sterilized at 120°C for 15 minutes. This medium was inoculated with Porodisculus pendulus NK-729W strain (FERN BP-1860) and Coriolus versicolor IFO 30340 and Phanerochaete chrysosporium ATCC 34541 known so far as lignin-degrading microorganism as control,respectively,and cultured as 28°C for one month.

After cultivation, Klason lignin (JIS p8008-1961) and acid-soluble lignin (Chemistry of Lignin, edited by Junzo Nakano, p 53 (1982)) in wood powder were determined quantitatively. The weight loss was also measured to calculate the amount of carbohydrates. The result is shown in Table 2.

6

EP 0 295 063 B1

Table 2 : Result of wood-degradation

| Wood powder | | Before cultivation | After cultivation | | |
|---|---|---|---|---|---|
| | | | Novel microorganism of the invention | Coriolus versicolor | Phanerochaete chrysosporium |
| Weight loss | (g) | | 0.1463 | 0.0825 | 0.0452 |
| Klason lignin content | (g) | 0.2237 | 0.1091 | 0.1914 | 0.1998 |
| Acid-soluble lignin content | (g) | 0.0314 | 0.0374 | 0.0325 | 0.0367 |
| Total lignin content | (g) | 0.2551 | 0.1465 | 0.2239 | 0.2365 |
| Carbohydrate content | (g) | 0.7449 | 0.7072 | 0.6936 | 0.7183 |
| Klason lignin loss | (KL,%) | | 51.23 | 14.44 | 10.68 |
| Carbohydrate loss | (C, %) | | 5.06 | 6.89 | 3.57 |
| KL/C | | | 10.12 | 2.10 | 2.99 |

As apparent from the result in Table 2, the novel microoraganism according to the present invention is outstandingly excellent in the lignin-degrading activity and has extremely high selectivity.

As described above, the present invention has succeeded in the development of novel microorganisms excellent in the lignin-degrading activity and capable of selectively degrading lignin.

Accordingly, since the microorganisms according to the present invention have excellent property of acting on lignin and degrading it or reducing the molecular weight therefor, they can be used effectively in the art of utilizing cellulose-containing biomass by lignin-degradation thereby improving the effect of cellulase as the pre-treatment to the saccharification of lignocellulose such as wood.

Furthermore, the present invention is also applicable to pulp and paper manufacturing process. That is, it can be utilized effectively also to the degradation or molecular weight reduction of lignin in the pulping, pulp bleaching and waste water treatment process.

It can be applied to all of other technical fields of degrading lignin or reducing the molecular weight thereof.

Furthermore, since the microorganisms of the new strain according to the present invention have extremely high lignin-degrading activity and selectively, they can be utilized for the industrial mass processing in each of the application uses and, accordingly, they are quite novel and can provide outstanding effect different from the known microorganisms.

As has been described above, industrialization of the lignin biodegradation has been made possible for the first time by the present invention.

## Claims

1. Microorganism strain FERM BP-1859 (NK-1148) having excellent lignin-degrading activity and capable of selectively degrading lignin.

2. Use of the strain according to claim 1 in a method for degradation of lignin.

3. Strain FERM BP-1860 (NK-729W) belonging to Porodisculus pendulus, having excellent lignin-degrading activity and capable of selectively degrading lignin.

4. Use of the strain according to claim 3 in a method for degradation of lignin.

**Patentansprüche**

1. Mikroorganismusstamm FERM BP-1859 (NK-1148) mit ausgezeichneter Ligninabbauaktivität und zu selektivem Ligninabbau fähig.

2. Verwendung des Stammes nach Anspruch 1 in einem Verfahren zum Abbauen von Lignin.

3. Zu Porodisculus pendulus gehörender Stamm FERM BP-1860 (NK-729W) mit ausgezeichneter Ligninabbauaktivität und zu selektivem Abbau von Lignin fähig.

4. Verwendung des Stammes nach Anspruch 3 bei einem Verfahren zum Abbauen von Lignin.

**Revendications**

1. Souche de micro-organisme FERM BP-1859 (NK-1148) ayant une excellente activité de dégradation de la lignine et capable de dégrader sélectivement de la lignine.

2. Utilisation de la souche selon la revendication 1, dans un procédé pour la dégradation de lignine.

3. Souche FERM BP-1860 (NK-729W) appartenant à l'espèce Porodisculus pendulus, ayant une excellente activité de dégradation de la lignine et capable de dégrader sélectivement de la lignine.

4. Utilisation de la souche selon la revendication 3, dans un procédé pour la dégradation de lignine.